# EUROPÄISCHE PATENTANMELDUNG

(11) **EP 1 518 569 A1**
(43) Veröffentlichungstag der Anmeldung: **30.03.2005**
(21) Anmeldenummer: 04020126.1
(22) Anmeldetag: 25.08.2004
(51) Int. Cl.: A61L 27/46, A61L 27/12, A61L 27/26, A61L 27/56

(54) **Implantatmaterial für den Knochen-Knorpel-Ersatz**

(30) Priorität: 27.08.2003 DE 10339953
(71) Anmelder: Coripharm Medizinprodukte GmbH & Co. KG., 64807 Dieburg (DE)
(72) Erfinder: Vogt, Sebastian Dr., 99084 Erfurt (DE); Schnabelrauch, Matthias, Dr., 07749 Jena (DE); Wilke, Ingo, Dr., 37133 Friedland (DE); Möller, Stephanie, Dr., 07749 Jena (DE); Dingeldein, Elvira, Dr., 63303 Dreieich (DE); Seidel, Peter, Dr., 64807 Dieburg (DE)
(74) Vertreter: Bock, Gerhard, Dipl.-Ing.

(57) **Zusammenfassung**

Die Aufgabe der Erfindung, ein Implantatmaterial für den Knochen-Knorpel-Ersatz bereitzustellen, das unter Nutzung der Pressfit-Technik sicher und mechanisch fest im Knochen implantiert werden kann und das unter in vitro-Bedingungen mit Osteoblasten und mit Chondrozyten selektiv besiedelt werden kann, wird dadurch gelöst, dass ein Implantatmaterial für den Knochen-Knorpel-Ersatz aus drei mit einander verbundenen, übereinanderliegenden Schichten A, B und C besteht, wobei die Schicht A ein interkonnektierendes Porensystem besitzt, die Schicht B eine porenfreie, geschlossene Schicht ist, und die Schicht C ein offen-poröse Schicht ist, deren Poren mit biokompatiblen und oder resorbierbaren Gel ausgefüllt sind, und daß die Schichten A, B und C aus einem Komposit, der aus mindestens einem vernetzten, resorbierbaren Makromer und einem anorganischen, resorbierbaren Füllstoff gebildet ist, bestehen.

Für die Verwendung des Implantatmaterials für den Knochen-Knorpel-Ersatz ist charakteristisch, daß im interkonnektierenden Porensystem der Schicht A Osteoblasten oder Stammzellen und im Porensystem der Schicht C Chondrozyten oder Stammzellen gleichzeitig unter in vitro Bedingungen kultiviert werden und daß nach erfolgter Kultivierung das Implantatmaterial für die Transplantation bereitgestellt wird.

## Beschreibung

Die vorliegende Erfindung betrifft ein Implantatmaterial für den Knochen-Knorpel-Ersatz, das eine effiziente Behandlung von Knochen-Knorpel-Defekten im Gelenkbereich ermöglichen soll.

Kombinierte Knorpel-Knochendefekte stellen eine sehr häufige Indikation in der Human- und Veterenärmedizin dar, welche die Mobilität und damit die Lebensqualität der Patienten stark beeinträchtigen.
Knorpeldefekte, der Verlust von Knorpelmaterial und beim Fortschreiten auch Schädigung des darunter liegenden Knochengewebes, werden durch degenerative Erkrankungen der Gelenke verursacht, wobei die Arthrose die am häufigsten auftretende Gelenkerkrankung ist.
Eine relativ erfolgreiche konventionelle Therapie beruht auf der Transplantation von autologen Knorpel-Knochen-Zylindern. Das Problem dabei besteht jedoch darin, daß nur sehr geringe Mengen dieser patienteneigenen Transplantate zugänglich sind. Insbesondere bei größeren zu versorgenden Knorpel-Defekten ist die ungenügende Verfügbarkeit ein schwerwiegendes Problem.
Der Knorpel wird durch Chondrozyten und der von ihnen gebildeten extrazellulären Matrix gebildet. Diese Matrix stellt ein kompliziertes dreidimensionales Gebilde dar, daß im wesentlichen aus vernetztem Kollagen II und Mucopolysacchariden besteht.
Auf Grundlage dieser Kenntnisse wurden zahlreiche Versuche unternommen, durch Methoden des s.g. Tissue Engineering Chondrozyten und ihre extrazelluläre Matrix unter in vitro-Bedingungen anzuzüchten und anschließend in die Knorpeldefekte zu implantieren um dadurch Knorpeldefekte behandeln zu können.

Ein grundlegendes Problem bei den bekannten technischen Lösungen besteht darin, daß artifizielles Knorpelgewebe mechanisch nicht genügend stabil im Knorpel-Defekt fixiert werden kann, so daß es den Druck- und Scherkräften im Gelenk widerstehen kann.

Der Erfindung liegt deshalb die Aufgabe zu Grunde, ein Implantatmaterial für den Knochen-Knorpel-Ersatz bereitzustellen, das unter Nutzung der Pressfit-Technik sicher und mechanisch fest im Knochen implantiert werden kann und das unter in vitro-Bedingungen mit Osteoblasten und mit Chondrozyten selektiv besiedelt werden kann. Dadurch soll erreicht werden, daß einerseits eine extrazelluläre Matrix durch die Chondrozyten unter in vitro-Bedingungen in einem definierten, vorherbestimmten Raum vorgebildet werden kann und dass andererseits zeitgleich durch Osteoblasten auch eine extrazelluläre Matrix ebenfalls in einem separaten, vorherbestimmten Raum vorgebildet wird. Durch die Implantation eines solchen, bereits besiedelten Materials soll erreicht werden, daß eine schnellere Integration des Konstrukts in das Knochengewebe möglich wird und daß die Chondrozyten mit der vorgebildeten extrazellulären Matrix mechanisch stabil fixiert sind. Ein weiteres Ziel besteht darin, daß das Implantat auch einen hinreichenden mechanischen Schutz für die Chondrozyten und ihrer extrazellulären Matrix vor mechanischer Beanspruchung, insbesondere vor Scherung, bieten soll.

Die Aufgabe wurde erfindungsgemäß durch eine Konstrukt gelöst, das aus drei mit einander verbundenen, übereinanderliegenden Schichten A, B und C gebildet wird, wobei die Schicht A ein interkonnektierendes Porensystem besitzt, die Schicht B eine porenfreie, geschlossene Schicht ist, und die Schicht C ein offen-poröse Schicht ist, deren Poren mit biokompatiblen und oder resorbierbaren Gel ausgefüllt sind, und daß die Schichten A, B und C aus einem Komposit aus mindestens einem vernetzten, resorbierbaren Makromer und oder einem vernetzten biokompatiblen Monomer und einem anorganischen, resorbierbaren Füllstoff gebildet sind.
Weiterhin ist erfindungsgemäß, dass das Konstrukt aus drei mit einander verbundenen, übereinanderliegenden Schichten A, B und C gebildet wird, wobei die Schicht A ein interkonnektierendes Porensystem besitzt, die Schicht B eine porenfreie, geschlossene Schicht ist, und die Schicht C eine offen poröse Schicht ist, deren Poren mit biokompatiblen und oder resorbierbaren Gel ausgefüllt sind, und daß die Schichten A, B und C aus einem Komposit aus mindestens einem vernetzten, resorbierbaren Makromer und oder einem vernetzten biokompatiblen Monomer und einem nanopartikulärem Hydroxylapatit gebildet sind.

Gemäß der Erfindung, sind die anorganischen Füllstoffe nanopartikuläres Hydroxylapatit, Tricalciumphosphat, Octacalciumphosphat, resorbierbares Phosphatglas, Calciumcarbonat, Magnesiumcarbonat und/oder Calciumsulfat-Dihydrat. Das bevorzugte nanopartikuläre Hydroxylapatit hat Kristallitlänge von ca. 50 nm und eine Kristallitbreite von ca. 20 nm.

Erfindungsgemäß ist, dass Methacrylat-terminierte und/oder Acrylatterminierte Oligoester, die aus Diolen oder Triolen oder Zuckeralkoholen oder Aminosäuren oder Diaminen oder Triaminen und α-Hydroxycarbonsäureestern aufgebaut sind, die resorbierbaren Makromere bilden. Weiterhin ist es im Sinne der Erfindung, Gemische aus vernetzten Methacrylat-terminiertem und/oder Acrylat-terminierten Oligoestern und resorbierbaren Polyestern, wie zum Beispiel Polylactiden, oder resorbierbaren Proteinen, wie zum Beispiel Kollagen und Gelatine, einzusetzen.

Gemäß der Erfindung sind die biokompatible Monomere niedermolekulare Methacrylate, Dimethacrylate, Trimethacrylate, Tetramethacrylate, Acrylate, Diacrylate, Triacrylate und Tetraacrylate. Im Sinne der Erfindung ist die bevorzugte Verwendung von Methacrylsäuremethylester, Methacrylsäurehydroxyethylester, Acrylsäuremethylester, Acrylsäurehydroxyethylester, Ethylenglykoldimethacrylat, Ethylengykoldiacrylat, Diethylenglykoldimethacrylat, Diethylenglykolacrylat, Triethylenglykoldimethacrylat, Triethylenglykol-diacrylat, Glycerintrimethacrylat, Glycerintriacrylat, Penterythroltetra-acrylat und Pentaerythroltetraacrylat. Im Sinne der Erfindung ist ebenfalls die Verwendung von Itaconsäureestern. Weiterhin ist es auch möglich, Gemische aus vernetzten niedermolekularen Methacrylaten, Dimethacrylaten, Trimethacrylaten, Tetramethacrylaten, Acrylaten, Diacrylaten, Triacrylaten und Tetraacrylaten zu verwenden, die zusätzlich in den Monomeren lösliche Polymere, wie zum Beispiel Polymethmathacrylat, Polymethylacrylat, enthalten.

Gemäß der Erfindung, sind in den Schichten A, B und C 5 bis 95 Masseprozent anorganische Füllstoffe enthalten.
Es ist erfindungsgemäß, dass mittlere Porendurchmesser der interkonnektierenden Porensysteme der Schichten A und C von 50 µm bis 2000 µm bevorzugt sind. Die interkonnektierenden Porensysteme können durch Auslaugung oder durch Verspinnen von Makromer-Füllstoffgemischen hergestellt werden.
Dabei können die mittleren Porendurchmesser der interkonnektierenden Porensysteme der Schichten A und C gleich oder auch unterschiedlich sein.

Das biokompatible und/oder resorbierbare Gel ist aus Calciumalginat, Calciumpektinat, Kollagen, Gelatine, Chitosan, Hyaluronsäure, Polyvinylalkohol, Methacryloyl-glycidyl-dextran, Methacrylat-terminierte Polyethylenglykole, Methacrylat-terminierte Milchsäure-Polyethylen-glykol-Kopolymere, Carboxymethylhyaluronsäure, Carboxymethyl-cellulose und/oder Carboxymethyldextran sowie aus Chitosan- Komplexen und mindestens einem anionischen Polymer aus der Gruppe der Carboxymethylcellulose, Carboxymethylhyaluronsäure und des Carboxymethyldextrans.

Im Sinne der Erfindung ist die Verwendung des Implantatmaterials für den Knochen-Knorpel-Ersatz in der Weise, daß im interkonnektierenden Porensystem der Schicht A Osteoblasten oder deren Stammzellen und im Porensystem der Schicht C Chondrozyten oder deren Stammzellen gleichzeitig unter in vitro-Bedingungen kultiviert werden.

Es ist erfindungsgemäß, daß nach erfolgter Kultivierung von Osteoblasten oder deren Stammzellen in der Schicht A und von Chondrozyten oder deren Stammzellen in der Schicht C das Implantatmaterial zur Transplantation bereitgestellt wird.

Die erfindungsgemäße Verwendung des Implantatmaterials für den Knochen-Knorpel-Ersatz ist dadurch charakterisiert, dass es als Implantat eingesetzt wird, indem die Schicht A so im Gelenkknochen positioniert wird, dass die Schicht C im Knorbelbereich C des Gelenks gelegen ist.

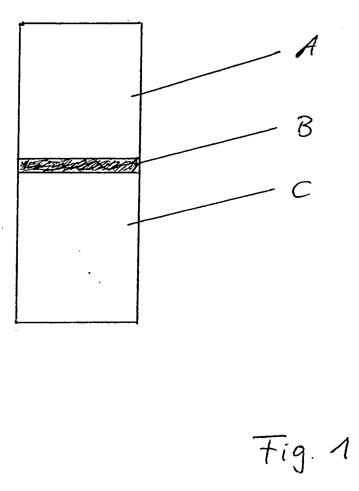

Die Erfindung soll an Hand des nachstehenden Beispiels näher erläutert werden, ohne jedoch die Erfindung hierauf einzuschränken:
Das Implantatmaterial ist ein Zylinder mit einem Durchmesser von 10 mm und einer Höhe von 10 mm und besteht aus drei übereinanderliegenden, mit einander verbundenen Schichten A, B und C.
Die Schicht A hat eine Höhe von 4,5 mm und enthält ein interkonnektierendes Porensystem mit einem mittleren Porendurchmesser von 250 µm. Die Porosität der Schicht A beträgt 69 Prozent.
Die an die Schicht A sich anschließende Schicht B ist nicht porös und hat eine Höhe von 1 mm.
An diese Schicht schließt sich die Schicht C an. Die Schicht C hat eine Höhe von 4,5 mm und enthält ein interkonnektierendes Porensystem mit einem mittleren Porendurchmesser von 250 µm.
Die Schichten A, B und C bestehen aus einem Komposit, der aus 50 Masseprozent nanopartikulärem Hydroxylapatit (Länge der Kristalle ca. 50 nm, Breite der Kristalle ca. 20 nm) und 50 Masseprozent einer Matrix aus einem vernetzten Makromer gebildet ist.
Dieses Makromer ist ein radikalisch vernetzbarer Methacrylatterminiertes Oligoester. Dieser Oligoester wird durch Ringöffnungsoligomerisation von L-Lactid unter Verwendung von Triethylenglykol als Starter in Gegenwart von Zinn(II)-2-ethylhexanoat synthetisiert. Das Stoffmengenverhältnis von Triethylenglykol zu L-Lactid beträgt dabei 1 : 4.
Die beiden terminalen Hydroxylgruppen des Oligoesters werden mit Methacrylsäurechlorid in Gegenwart von Triethylamin verestert.
Alle in der Beschreibung, den nachfolgenden Ansprüchen und der Zeichnung dargestellten Merkmale können sowohl einzeln als auch in beliebiger Kombination miteinander erfindungswesentlich sein.

## Patentansprüche

1. Implantatmaterial für den Knochen-Knorpel-Ersatz bestehend aus drei mit einander verbundenen, übereinanderliegenden Schichten A, B und C, wobei die Schicht A ein interkonnektierendes Porensystem, die Schicht B eine porenfreie, geschlossene Schicht und die Schicht C ein offen-poröse Schicht ist.

2. Implantatmaterial für den Knochen-Knorpel-Ersatz gemäß Anspruch 1, **dadurch gekennzeichnet, dass** die Poren der Schicht C mit biokompatiblen und/oder resorbierbaren Gel ausgefüllt sind.

3. Implantatmaterial für den Knochen-Knorpel-Ersatz gemäß Anspruch 1, **dadurch gekennzeichnet, dass** die Schichten A, B und C aus einem Komposit bestehen, der aus mindestens einem vernetzten, resorbierbaren Makromer und/oder vernetzten biokompatiblen Monomer sowie einem anorganischen, resorbierbaren Füllstoff gebildet ist.

4. Implantatmaterial für den Knochen-Knorpel-Ersatz gemäß Anspruch 1, **dadurch gekennzeichnet, dass** die Schichten A, B und C aus einem Komposit bestehen, der aus mindestens einem vernetzten, resorbierbaren Makromer und/oder einem vernetzten biokompatiblen Monomer und nanopartikulärem Hydroxylapatit gebildet ist.

5. Implantatmaterial für den Knochen-Knorpel-Ersatz gemäß den Ansprüchen 1 und 3, **dadurch gekennzeichnet, dass** die anorganischen Füllstoffe nanopartikuläres Hydroxylapatit, Tricalciumphosphat, Octacalciumphosphat, resorbierbares Phosphatglas, Calciumcarbonat, Magnesiumcarbonat und/oder Calciumsulfat-Dihydrat sind.

6. Implantatmaterial für den Knochen-Knorpel-Ersatz gemäß den Ansprüchen 1 und 3, **dadurch gekennzeichnet, dass** die resorbierbaren Makromere Methacrylat-terminierte und/oder Acrylatterminierte Oligoester, die aus Diolen oder Triolen oder Zuckeralkoholen oder Aminosäuren oder Diaminen oder Triaminen und α-Hydroxycarbonsäureestern sind.

7. Implantatmaterial für den Knochen-Knorpel-Ersatz gemäß den Ansprüchen 1 und 3, **dadurch gekennzeichnet, dass** die biokompatible Monomere niedermolekulare Methacrylate, Dimethacrylate, Trimethacrylate, Tetramethacrylate, Acrylate, Diacrylate, Triacrylate und Tetraacrylate bevorzugt sind.

8. Implantatmaterial für den Knochen-Knorpel-Ersatz gemäß den Ansprüchen 1, 3 und 5, **dadurch gekennzeichnet, dass** in den Schichten A, B und C 5 bis 95 Masseprozent anorganische Füllstoffe bevorzugt enthalten sind.

9. Implantatmaterial für den Knochen-Knorpel-Ersatz gemäß Anspruch 1, **dadurch gekennzeichnet, dass** der mittlere Porendurchmesser der interkonnektierenden Porensysteme der Schichten A und C 50 µm bis 2000 µm beträgt.

10. Implantatmaterial für den Knochen-Knorpel-Ersatz gemäß Anspruch 9, **dadurch gekennzeichnet, dass** die mittleren Porendurchmesser der interkonnektierenden Porensysteme der Schichten A und C gleich oder unterschiedlich sind.

11. Implantatmaterial für den Knochen-Knorpel-Ersatz gemäß den Ansprüchen 1 und 3, **dadurch gekennzeichnet, dass** das biokompatible und/oder resorbierbare Gel Calciumalginat, Calciumpektinat, Kollagen, Gelatine, Chitosan, Hyaluronsäure, Polyvinylalkohol, Methacryloyl-glycidyl-dextran, Methacrylat-terminierte Polyethylen-glykole, Methacrylat-terminiert Milchsäure-Polyethylen-glykol-Kopolymere, Carboxymethylhyaluronsäure, Carboxymethylcellulose, Carboxymethyldextran ist und Komplexe aus Chitosan und/oder Kollagen und mindestens einem anionischen Polymer aus der Gruppe der Carboxymethylcellulose, Carboxymethylhyaluronsäure und des Carboxymethyldextrans enthält.

12. Verwendung des Implantatmaterials gemäß einem oder mehrerer der voranstehenden Ansprüche für den Knochen-Knorpel-Ersatz.

13. Verwendung des Implantatmaterials gemäß Anspruch 12, **dadurch gekennzeichnet, dass** im interkonnektierenden Porensystem der Schicht A Osteoblasten oder deren Stammzellen und im Porensystem der Schicht C Chondrozyten oder deren Stammzellen gleichzeitig unter in vitro Bedingungen kultiviert werden.

14. Verwendung des Implantatmaterials gemäß Anspruch 13, **dadurch gekennzeichnet, dass** nach erfolgter Kultivierung von Osteoblasten oder Stammzellen in der Schicht A und von Chondrozyten oder Stammzellen in der Schicht C das Implantatmaterial zur Transplantation eingesetzt wird.

15. Verwendung des Implantatmaterials gemäß Anspruch 14, **dadurch gekennzeichnet, dass** es mit der Schicht A so im Gelenkknochen positioniert wird, dass die Schicht C im Knorbelbereich des Gelenks gelegen ist.
